(19) Europäisches Patentamt

European Patent Office

Office européen des brevets

(11) **EP 1 541 165 A1**

(12) **EUROPEAN PATENT APPLICATION**
published in accordance with Art. 158(3) EPC

(43) Date of publication:
**15.06.2005 Bulletin 2005/24**

(51) Int Cl.⁷: **A61K 38/00**, A61K 9/08,
A61J 1/00

(21) Application number: **03791289.6**

(22) Date of filing: **26.08.2003**

(86) International application number:
**PCT/JP2003/010754**

(87) International publication number:
**WO 2004/019966 (11.03.2004 Gazette 2004/11)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **27.08.2002 JP 2002247298**

(71) Applicant: **CHUGAI SEIYAKU KABUSHIKI
KAISHA
Tokyo, 115-8543 (JP)**

(72) Inventors:
• **TANIKAWA, Masahiko, Chugai Seiyaku K. K.
Tokyo 171-8545 (JP)**
• **OMURA, Takeshi, Chugai Seiyaku K. K.
Tokyo 171-8545 (JP)**

(74) Representative: **VOSSIUS & PARTNER
Siebertstrasse 4
81675 München (DE)**

(54) **METHOD OF STABILIZING PROTEIN SOLUTION PREPARATION**

(57) Problems to be Solved: The present invention provides a method for improving the stability of protein solution formulations by suppressing the formation of associated matter from physiologically active proteins (e.g., antibodies, enzymes, hormones, cytokines) in a solution form.

Means for Solving the Problems: A method for stabilizing a protein solution formulation, which comprises storing the protein solution formulation under magnetic field lines, as well as a storage container for holding a protein solution formulation, which is equipped with a magnetic field generator.

EP 1 541 165 A1

## EP 1 541 165 A1

**Description**

<u>TECHNICAL FIELD</u>

The present invention relates to a method for stabilizing protein solution formulations and to a storage container for holding protein solution formulations. More specifically, the present invention relates to a method for stabilizing physiologically active protein formulations by storing protein solution formulations under magnetic field lines, as well as to a magnetic field generator-equipped storage container for holding protein solution formulations. The method and storage container of the present invention suppress the association of physiologically active proteins and are particularly useful for stabilizing protein solution formulations.

<u>BACKGROUND ART</u>

Advances in gene recombination technology have enabled a stable supply of various physiologically active protein formulations. Most of these physiologically active proteins are known to be associated in an aqueous solution, which constitutes a major cause of reducing the stability of these formulations. To ensure the stability of these formulations, they are provided as lyophilized formulations or in the form of protein solution formulations supplemented with various additives for improving the stability.

For example, it has been found that stabilization effects are obtained by addition of stabilizers including high-molecular weight materials such as proteins (e.g., human serum albumin, purified gelatin) or low-molecular weight materials such as polyols, amino acids and surfactants. However, when added as stabilizers, organism-derived high-molecular weight materials such as proteins are disadvantageous in that very complicated processes are necessary to remove contaminants, such as viruses, originating from the stabilizers per se. Also, when heat treatment is carried out for the purpose of virus inactivation, such stabilizers may cause problems such as association and aggregation under heat stress.

For this reason, there has been a strong demand for a method for stabilizing protein solution formulations, which minimizes the amounts of stabilizers and allows suppression of protein molecule association through simple treatment.

The object of the present invention is to provide a method for improving the stability of protein solution formulations by suppressing the formation of associated matter from molecules such as physiologically active proteins (e.g., antibodies, enzymes, hormones, cytokines) in a solution form.

<u>DISCLOSURE OF THE INVENTION</u>

As a result of extensive and intensive efforts made to achieve the above object, the inventors of the present invention have unexpectedly found that the formation of associated matter (e.g., dimers) can be significantly suppressed when protein solution formulations are placed under magnetic field lines. This finding led to the completion of the present invention.

Namely, the present invention provides the following:

(1) a method for stabilizing a protein solution formulation, which comprises storing the protein solution formulation under magnetic field lines;
(2) the method according to (1) above, wherein the magnetic flux density is 1 mT (millitesla) or more;
(3) the method according to (1) or (2) above, wherein the protein is a physiologically active protein;
(4) the method according to (3) above, wherein the physiologically active protein is selected from an antibody, an enzyme, a cytokine and a hormone;
(5) the method according to (4) above, wherein the physiologically active protein is a hematopoietic factor;
(6) the method according to (5) above, wherein the hematopoietic factor is erythropoietin or granulocyte colony-stimulating factor;
(7) the method according to (4) above, wherein the physiologically active protein is an antibody;
(8) the method according to (1) above, wherein the protein solution formulation is in the form of a pre-filled syringe formulation;
(9) a storage container for holding a protein solution formulation, which comprises a magnetic field generator;
(10) the storage container according to (9) above, wherein the magnetic field generator is a magnet;
(11) a method for stabilizing a protein-containing solution, which comprises storing the protein-containing solution under magnetic field lines;
(12) the method according to (11) above, wherein the protein-containing solution is a bulk solution for protein production;

(13) a stabilized protein formulation, which is prepared from a bulk solution for protein production stored under magnetic field lines;

(14) the formulation according to (13) above, wherein the stabilized protein formulation is in the form of a solution formulation;

(15) the formulation according to (13) above, wherein the stabilized protein formulation is in the form of a lyophilized formulation;

(16) a method for suppressing the formation of associated matter in a protein solution formulation, which comprises storing the protein solution formulation under magnetic field lines; and

(17) the use of a magnetic field generator for stabilization of a protein solution formulation.

BRIEF DESCRIPTION OF DRAWINGS

Figure 1 shows a schematic view of an accelerated test for injectable EPO formulations under magnetic field lines.
Figure 2 shows an embodiment for storing solution formulations according to the method of the present invention (a: side view; b: top view).
Figure 3 shows a side view of an embodiment for storing solution formulations according to the method of the present invention.
Figure 4 shows an embodiment for storing G-CSF solutions under magnetic field lines.

BEST MODE FOR CARRYING OUT THE INVENTION

Proteins used in the present invention are preferably, but not limited to, physiologically active proteins. The protein solution of the present invention is not limited to pharmaceutical use, and it is therefore intended to provide a method for stabilizing any protein-containing solutions in which protein stabilization is required. Such proteins are contained in pharmaceuticals, foods, cosmetics and the like. Physiologically active proteins include, but are not limited to, antibodies, enzymes, cytokines and hormones. Specific examples include, but are not limited to, hematopoietic factors such as granulocyte colony-stimulating factor (G-CSF), granulocyte-macrophage colony-stimulating factor (GM-CSF), erythropoietin (EPO) and thrombopoietin, cytokines such as interferons, IL-1 and IL-6, immunoglobulins such as monoclonal antibodies and humanized antibodies, tissue plasminogen activator (TPA), urokinase, serum albumin, blood coagulation factor VIII, leptin, insulin, and stem cell growth factor (SCF). Among these physiologically active proteins, preferred are hematopoietic factors such as EPO, G-CSF, GM-CSF and thrombopoietin, and more preferred are EPO and G-CSF. Also preferred as physiologically active proteins are immunoglobulins, and more preferred are monoclonal antibodies and humanized antibodies.

The physiologically active protein as used in the stabilization method of the present invention is intended to mean a protein having substantially the same biological activities as a corresponding physiologically active protein of mammalian (especially human) origin. Such a protein may either be native or genetically recombinant, preferably genetically recombinant. Genetically recombinant and physiologically active proteins may be prepared by production in bacterial cells such as *E. coli*; yeast cells; or animal-derived cultured cells such as Chinese hamster ovary (CHO) cells, C127 cells or COS cells. The proteins thus prepared are isolated and purified in various manners before use. Such genetically recombinant proteins encompass those having the same amino acid sequence as the corresponding native protein, as well as those comprising deletion, substitution or addition of one or more amino acids in the amino acid sequence, but retaining the biological activities mentioned above. Further, such proteins include those chemically modified with PEG, etc.

Examples of a physiologically active protein include glycoproteins having sugar chains. Although the origin of sugar chains is not limited in any way, preferred are sugar chains attached to mammalian cells. Mammalian cells include, for example, Chinese hamster ovary (CHO) cells, BHK cells, COS cells and human-derived cells, with CHO cells being most preferred.

When a physiologically active protein is G-CSF, any G-CSF can be used as long as it is highly purified. G-CSF as used herein may be prepared in any manner; for example, by culturing human tumor cell lines or by genetically engineered production in bacterial cells such as *E. coli*; yeast cells; or animal-derived cultured cells such as Chinese hamster ovary (CHO) cells, C127 cells or COS cells. G-CSF thus prepared is extracted, isolated and purified in various manners before use. Preferred are those produced by genetic recombination techniques in *E. coli* cells, yeast cells or CHO cells. The most preferred are those produced recombinantly in CHO cells. In addition, G-CSF may be chemically modified with PEG, etc. (see International Publication No. WO90/12874).

When a physiologically active protein is EPO, it may be prepared in any manner. Examples of EPO in the present invention include those derived from human urine as well as those produced in Chinese hamster ovary (CHO) cells, BHK cells, COS cells, human-derived cells or the like (e.g., as described in JP KOKAI 61-12288) by genetic engineering techniques. EPO thus prepared is extracted, isolated and purified in various manners before use. In addition, EPO may be chemically modified with PEG, etc. (see International Publication No. WO90/12874). EPO as used herein further includes unglycosylated EPO that is chemically modified with PEG, etc. Likewise, EPO analogs are also included, which are modified to have at least one additional site for N-linked or O-linked glycosylation in the amino acid sequence of EPO (see, e.g., JP KOKAI 08-151398, JP KOHYO 08-506023). Instead of increasing the number of glycosylation sites, EPO analogs may also be modified to have an increased content of sialic acid for attaining additional sugar chains.

When a physiologically active protein is a monoclonal antibody, it may be prepared in any manner. In general, a monoclonal antibody can be produced using known techniques by immunizing a sensitizing antigen in accordance with conventional procedures for immunization, fusing the resulting immunocytes with known parent cells through conventional procedures for cell fusion, and then screening monoclonal antibody-producing cells through conventional procedures for screening.

Alternatively, antibody genes are cloned from hybridomas, integrated into appropriate vectors, and then transformed into hosts to produce antibody molecules using gene recombination technology. The genetically recombinant antibodies thus produced may also be used in the present invention (see, e.g., Carl, A. K. Borrebaeck, James, W. Larrick, THERAPEUTIC MONOCLONAL ANTIBODIES, Published in the United Kingdom by MACMILLAN PUBLISHERS LTD, 1990).

More specifically, cDNA of antibody variable domains (V domains) is synthesized from hybridoma mRNA using reverse transcriptase. Upon obtaining DNA encoding the target antibody V domains, the DNA is ligated to DNA encoding desired antibody constant domains (C domains) and integrated into an expression vector. Alternatively, the DNA encoding the antibody V domains may be integrated into an expression vector carrying the DNA of the antibody C domains. The DNA construct is integrated into an expression vector such that it is expressed under control of an expression regulatory region, e.g., an enhancer or a promoter. Host cells are then transformed with this expression vector for antibody expression.

In the present invention, it is possible to use genetically recombinant antibodies (e.g., chimeric antibodies and humanized antibodies) that are artificially modified with a view to attenuating the characteristics as heteroantigen to human. These modified antibodies may be prepared in a known manner. A chimeric antibody is composed of variable domains of heavy and light chains from a non-human mammalian (e.g., mouse) antibody and constant domains of heavy and light chains from a human antibody.

To obtain chimeric antibodies, DNAs encoding such mouse antibody variable domains may be ligated to DNAs encoding the human antibody constant domains, and then integrated into an expression vector, followed by transformation into a host for antibody production.

Humanized antibodies are also called reshaped human antibodies and are obtained by grafting complementarity determining regions (CDRs) of non-human mammalian (e.g., mouse) antibodies to replace those of human antibodies. Standard gene recombination procedures for this purpose are also known. More specifically, a DNA sequence is designed to allow ligation between CDRs of mouse antibody and framework regions (FRs) of human antibody, and is synthesized by PCR from several oligonucleotides which are prepared so as to have sections overlapping with one another at the ends. The DNA thus obtained is ligated to DNA encoding human antibody constant domains, and integrated into an expression vector, followed by transformation into a host for antibody production (see European Patent Publication No. EP 239400 and International Publication No. WO 96/02576). The FRs of human antibody, which is ligated via CDRs, are selected such that the complementarity determining regions form a favorable antigen-binding site. If necessary, amino acid substitution(s) may be made in the framework regions of antibody variable domains such that the complementarity determining regions of reshaped humanized antibody may form an appropriate antigen-binding site (Sato, K. et al., Cancer Res. (1993) 53, 851-856).

A humanized anti-IL-6 receptor antibody (hPM-1) can be presented as a preferred example for such reshaped humanized antibodies (see International Publication No. WO92-19759). In addition, a humanized anti-HM1.24 antigen monoclonal antibody (see International Publication No. WO98-14580), a humanized anti-parathyroid hormone-related peptide antibody (anti-PTHrP antibody; see International Publication No. WO98-13388), a humanized anti-tissue factor antibody (see International Publication No. W099-51743) and the like are also preferred for use in the present invention.

Procedures for obtaining human antibodies are also known. For example, human lymphocytes are sensitized *in vitro* with a desired antigen or a desired antigen-expressing cell, and the sensitized lymphocytes are then fused with human myeloma cells (e.g., U266) to give desired human antibodies having binding activity to the antigen (see JP KOKOKU 01-59878). Alternatively, transgenic animals having the entire repertories of human antibody genes may be immunized with an antigen to obtain desired human antibodies (see International Publication Nos. WO 93/12227, WO 92/03918, WO 94/02602, WO 94/25585, WO 96/34096 and WO 96/33735). There are another techniques using human antibody libraries to give human antibodies by panning. For example, human antibody variable domains may each be expressed by phage display technology as a single-chain antibody (scFv) on the surface of phages, followed by selection of phages binding to the antigen. When genes of the selected phages are analyzed, it is possible to determine DNA sequences encoding human antibody variable domains binding to the antigen. Once the DNA sequences of scFv binding to the antigen have been identified, the sequences may be used to construct appropriate expression vectors to obtain human antibodies. These techniques are already well known and can be found in WO 92/01047, WO 92/20791, WO 93/06213, WO 93/11236, WO 93/19172, WO 95/01438 and WO 95/15388.

In another embodiment, human antibodies produced in transgenic animals and the like are also preferred.

Furthermore, the antibody as used herein encompasses antibody fragments including Fab, (Fab')$_2$, Fc, Fc' and Fd, as well as reshaped antibodies including monovalent or multivalent single chain antibodies (scFV).

As used herein, the term "physiologically active protein-containing sample" is intended to mean a sample containing any protein, whether native or recombinant. It is preferably a culture medium of mammalian cells (e.g., CHO cells) containing physiologically active protein molecules produced by culture, which may further be subjected to partial purification or other certain treatment(s).

If desired, a physiologically active protein solution formulation may further comprise, in addition to a physiologically active protein, other ingredients such as a stabilizer (e.g., surfactants, amino acids), a diluent, a solubilizer, an excipient, a pH adjuster, a soothing agent, a buffering agent, a sulfur-containing reducing agent and an antioxidant. Such a formulation may be prepared by dissolving these ingredients in a commonly used buffer.

Physiologically active protein solution formulations can usually be provided in containers having a definite volume, including sealed and sterilized plastic or glass vials, ampoules and syringes, as well as in large volume containers like bottles. The stabilization method of the present invention can be used for solution formulations contained in any of these containers. Preferred are solution formulations in the form of pre-filled syringe formulations.

In addition, vials are also presented as a preferred example.

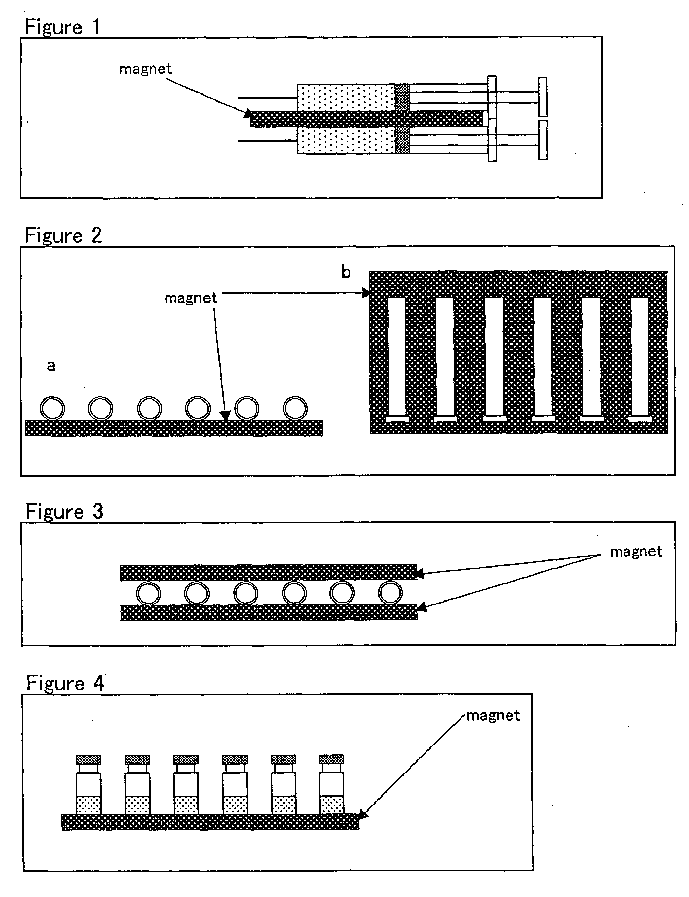

In the present invention, solution formulations of physiologically active proteins are stored under magnetic field lines. Examples of a magnetic field generator include, but are not limited to, a magnet, a magnetic head produced by semiconductor processes, and an array of microcoils through which an electric current is passed. Preferably, containers of the solution formulations are surrounded by magnets because they are inexpensive and easy to use. Figure 1 shows an embodiment of the present invention. Although such a magnet may be of any shape and may be placed in any orientation, a sheet-shaped magnet is preferred in view of the packaged form of the solution formulations. Also, taking into account the fact that the intensity of magnetic field lines depends on the distance from the magnet surface as well as the demand that drug solutions in syringes should be distributed as uniformly as possible, a magnet(s) is preferably placed such that the S-N direction is perpendicular to the longitudinal axis of the syringes (see Figure 2). One or more magnetic field generators are arranged in such a manner that physiologically active protein solution formulations to be held are placed under magnetic field lines. Syringes may be arranged on one sheet-shaped magnet as shown in Figure 2 or may be arranged between two sheet-shaped magnets as shown in Figure 3.

Alternatively, vials may be arranged on a sheet-shaped magnet as shown in Figure 4.

Physiologically active protein solution formulations and a magnet(s) may be placed in any arrangement as long as the solution formulations are stored under magnetic field lines. The magnetization of the magnet(s) may be oriented in any direction as long as the solution formulations are stored under magnetic field lines. For example, in Figure 1, the magnet may be placed in such a manner that the upper surface is N pole and the lower surface is S pole, or vice versa. In Figures 2 and 4, the surface contacted with syringes or vials may be either N pole or S pole. In Figure 3, one of two surfaces contacted with syringes may be N pole and the other S pole, or alternatively both of them may be the same pole.

The magnetic flux density (i.e., the intensity of magnetic field) is 1 mT or more, preferably 1 to 450 mT, and more preferably 1 to 150 mT.

The present invention further provides a storage container for holding physiologically active protein solution formulations, which is equipped with a magnetic field generator. Although the storage container of the present invention may be of any shape and may be of any material, it has a site(s) for holding the above-mentioned magnetic field generator(s) and a solution formulation(s). The storage container may be a storage box, a refrigerator, a transport container or the like.

The present invention further provides a method for stabilizing a protein bulk solution, which comprises storing the protein bulk solution under magnetic field lines. The present invention also enables the preparation of stabilized protein formulations from a protein bulk solution stored under magnetic field lines. Such stabilized protein formulations may be either in the form of solution formulations or lyophilized formulations.

When stored according to the method of the present invention, physiologically active protein solution formulations have been found to suppress the formation of associated matter even after long-term storage. For example, after injectable erythropoietin (EPO) formulations (750 IU) were subjected to an accelerated test in condition that they were stored at 40°C for 2 weeks and 1 month in the absence of magnetic field lines, the formulations were evaluated by purity test (SDS-PAGE) and quantification test (liquid chromatography). As a result, they were found to show increased amounts of dimers in the purity test and reduced levels of EPO content in the quantification test. For comparison, the same purity test (SDS-PAGE) and quantification test (liquid chromatography) were performed on injectable EPO formulations (750 IU) after they were subjected to an accelerated test in the condition that they stored at 40°C for 2 weeks and 1 month under magnetic field lines (about 100 mT). The results indicate that there was no change in any property to be evaluated when compared to unaccelerated formulations, except that the formulations accelerated at 40°C for 1 month showed slightly increased amounts of dimers in the purity test.

Without being bound by any particular theory, the inventors of the present invention estimate the mechanism of the present invention as follows. Namely, it is generally believed that protein association may be caused by binding between hydrophobic moieties of protein molecules, which are exposed at the surface of their three-dimensional structure due to some influence (e.g., heat or collisions with the container surface). Thus, if protein molecules in an aqueous solution can be restrained by some force, random collisions of protein molecules can be prevented. In the present invention, it is believed that protein molecules in an aqueous solution are arranged regularly under a strong magnetic field and hence are restrained to prevent their random collisions, thus suppressing their association caused by binding between hydrophobic moieties of the molecules.

The method and storage container of the present invention are therefore based on a technical idea completely different from the conventional approach in which stabilizers are added to stabilize physiologically active protein formulations. The present invention suppresses the formation of associated matter through a simple process and also enables long-term storage of the formulations. The method and the storage container of the present invention can be used to reduce the amount of additives to be used.

The present invention will now be further described in the following examples, which are not intended to limit the scope of the invention. Based on the detailed description, various changes and modifications will be apparent to those skilled in the art, and such changes and modifications fall within the scope of the invention.

EXAMPLES

Example 1: Effects in EPO formulations

1) Samples

Injectable EPO formulations (750 IU) were used (syringe formulations, EPO concentration: 1500 IU/ml, volume: 0.5 ml, Chugai Pharmaceutical Co., Ltd.).

2) Test procedures

Injectable EPO formulations (750 IU) were provided with a magnet (about 100 mT) (see Figure 1) and stored in a thermostatic chamber (40°C) for 2 weeks and 1 month before being evaluated. In Figure 1, the sheet-shaped magnet was placed in such a manner that the upper surface was N pole and the lower S pole.

Simultaneously, samples were subjected to an accelerated test in the absence of magnetic field lines (<1 mT) at 40°C for 2 weeks and 1 month. The samples accelerated as above as well as unaccelerated samples were provided for evaluation. Each sample was evaluated by changes in EPO content, dimer content and other relative substance content as compared to the unaccelerated samples.

3) Evaluation procedures

3-1) Purity test (SDS-gel electrophoresis)

The samples prepared above are each used as a sample solution. Exactly 60 μL of each sample solution is taken and supplemented with non-reducing Sample Buffer[*1] (exactly 20 μL), followed by heating in a warm bath (50°C) for 15 minutes. After heating, BPB Solution[*2] (exactly 4.0 μL) is added to give an electrophoresis sample.

The electrophoresis samples and a molecular weight marker[*3] (7.5 μL each) are tested by electrophoresis under the following conditions and detected by Western blotting.

3-1-1) Electrophoresis

Apparatus: TEFCO electrophoresis unit
Gel: slab gel [gradient: 8% to 16%, 15 wells, 1.5 mm thickness, TEFCO]
Condition: constant current at 25 mA/gel

Duration: about 1.5 hours
Electrophoresis buffer: Electrophoresis Buffer*4

3-1-2) Western blotting

After electrophoresis, the gel is soaked in Transfer Buffer*5 (10 ml) and equilibrated for 30 minutes. Next, a sponge and a sheet of filter paper (first transfer filter paper, 20 cm $\times$ 10 cm, Pharmacia), both of which have been soaked in Transfer Buffer, are placed in sequence on the anode (+) side of a transfer unit and stacked with a ProBlot membrane*6. The ProBlot membrane has been soaked in methanol for about 30 seconds and further in Transfer Buffer for 5 minutes in advance. The equilibrated gel is placed on top of the membrane and further stacked with one sheet of filter paper and a sponge, both of which have been soaked in Transfer Buffer. The stack thus obtained is placed such that the membrane side faces the anode (+) and the gel side faces the cathode (-). The gel is transferred to the ProBlot membrane under the following conditions, followed by color development of the ProBlot membrane using anti-EPO rabbit serum.

Temperature: constant about 25°C
Condition: constant voltage at 65 $\pm$ 2 V
Duration: 120 minutes

* 1 Non-reducing Sample Buffer: Disodium ethylenediaminetetraacetate (74.48 mg) and sodium lauryl sulfate (5.0 g) are dissolve in Tris-HCl Solution*7 (50 ml).
* 2 BPB Solution: Tris-HCl Solution (0.625 ml), concentrated glycerin (3.5 ml) and bromphenol blue solution (5 mg) are mixed. The resulting solution is cold stored at 4°C.
* 3 Molecular weight marker solution: Prestained SDS-PAGE standard solution (Bio-Rad) or an equivalent thereof [containing Lysozyme (molecular weight: 20900), Soybean trypsin inhibitor (molecular weight: 29100), Carbonic anhydrase (molecular weight: 35500), Ovalbumin (molecular weight: 50600), Bovine serum albumin (molecular weight: 83000) and Phosphorylase B (molecular weight: 101000)]
* 4 Electrophoresis Buffer: Trishydroxymethylaminomethane (3.0 g) and glycine (14.4 g) are added to water (600 ml) and supplemented with sodium lauryl sulfate (1.0 g), followed by addition of water to 1000 ml.
* 5 Transfer Buffer: Trishydroxymethylaminomethane (6.0 g) and glycine (28.8 g) are added to water (600 ml) and supplemented with methanol (400 ml), followed by addition of water to 2000 ml.
* 6 ProBlot membrane: polyvinylidene difluoride membrane filter (8 cm $\times$ 8 cm, Applied Biosystems) or an equivalent thereof
* 7 Tris-HCl Solution: Trishydroxymethylaminomethane (3.0 g) is dissolved in water (30 ml) and adjusted to pH 6.8 with 1 mol/L hydrochloric acid, followed by addition of water to 100 ml. The resulting solution is cold stored at 4°C.

3-2) Quantification (liquid chromatography)

The samples prepared above are each used as a sample solution. A standard preparation of EPO is diluted to 1500 IU/ml with a 0.05% aqueous polysorbate 20 solution for use as a standard solution.
Exactly 100 μL each of each sample solution and the standard solution are tested by liquid chromatography under the following conditions for measurement of the peak areas of EPO ($A_T$ and $A_S$), respectively. The following equation is used to determine the EPO content (concentration, mg/ml) for each sample.

$$\text{EPO content (mg/ml)}$$

$$= \text{an amount of EPO (as EPO polypeptides) in EPO}$$

$$\text{standard preparation (mg/ml)} \times \frac{A_T}{A_S}$$

$A_T$: EPO peak area of sample solution
$A_S$: EPO peak area of standard solution

Operating conditions for liquid chromatography

Detector: UV absorptiometer (detection wavelength: 214 nm)
Column: a stainless steel tube (internal diameter: 5 mm, length: 25 cm) filled with about 5 $\mu$m butylsilylated silica gel (VYDAC 214TP54, Separations Group)
Column temperature: room temperature
Mobile phase: linear gradient of the following two solutions:

Solution A: a mixture of
water/acetonitrile/trifluoroacetic acid (400:100:1)
Solution B: a mixture of
acetonitrile/water/trifluoroacetic acid (400:100:1)

Gradient operation: The column is equilibrated with 35% Solution B and then injected with a sample solution or the standard solution. After maintaining 35% Solution B for 5 minutes, a linear gradient is run to 100% Solution B over 15 minutes. Then, 100% Solution B is maintained for 2 minutes.
Flow rate: adjusted such that the retention time of EPO is about 18 minutes.

4) Results

4-1) Purity test (SDS-PAGE)

The injectable EPO formulations (750 IU), which were subjected to an accelerated test under magnetic field lines at 40°C for 2 weeks and 1 month, were evaluated by SDS-PAGE.
As a result, the formulation accelerated in the absence of magnetic field lines at 40°C for 2 weeks was found to show an increased amount of dimers, whereas the formulation accelerated under magnetic field lines at 40°C for 2 weeks showed no change when compared to the unaccelerated formulation.
The formulations accelerated at 40°C for 1 month were found to show slightly increased amounts of dimers in both the presence and absence of magnetic field lines, but it was difficult to make a visual comparison of differences in the amount of dimers.

4-2) Quantification (liquid chromatography)

The injectable EPO formulations (750 IU), which were subjected to an accelerated test under magnetic field lines at 40°C for 2 weeks and 1 month, were evaluated by liquid chromatography. The results obtained are shown in Tables 1 and 2.
When compared to the unaccelerated formulation, the formulation accelerated in the absence of magnetic field lines at 40°C for 2 weeks was found to show a reduced level of EPO content, resulting in a residue level of 96%. Likewise, the formulation accelerated in the absence of magnetic field lines at 40°C for 1 month was found to show a significantly reduced level of EPO content, resulting in a residue level of 93%.
In contrast, the formulation accelerated under magnetic field lines at 40°C for 2 weeks showed almost no change in EPO content, and also the formulation accelerated at 40°C for 1 month was found to have a residue level of 97%. When taking into account the precision of this test (repeatability C.V. = 1.6%), there was no change in EPO content in either acceleration period.

Table 1

| Residue levels of EPO in formulations accelerated at 40°C for 2 weeks | | | |
|---|---|---|---|
| | Magnetic flux density | EPO conc. (of label) | Residue (%) |
| Unaccelerated | <1 mT | 93.8% | 100.0% |
| 40°C for 2 weeks | <1 mT | 89.7% | 95.6% |
| 40°C for 2 weeks | 100 mT | 93.7% | 99.9% |

Table 2

| Residue levels of epoetin beta in formulations accelerated at 40°C for 1 month | | | |
|---|---|---|---|
| | Magnetic flux density | EPO conc. (of label) | Residue (%) |
| Unaccelerated | <1 mT | 93.9% | 100.0% |
| 40°C for 1 month | <1 mT | 87.5% | 93.1% |
| 40°C for 1 month | 100 mT | 91.4% | 97.3% |

Example 2: Effects in G-CSF solutions

1) Sample preparation

A G-CSF solution (G-CSF concentration: about 0.5 mg/ml) was filtered through a disposable filter and filled into 5 ml glass vials in an amount of 1 ml per vial for use as test samples.

2) Storage conditions

These test samples were stored in a thermostatic chamber (30°C) in both the presence of magnetic field lines (about 100 mT[*8]) and the absence of magnetic field lines[*9] (see Figure 4). In Figure 4, the sheet-shaped magnet was placed in such a manner that the surface contacted with the vials was N pole and the other S pole.

*   8 Tesla (T): the unit of magnetic flux density, 1 tesla = 10,000 gauss (G)
*   9 In the absence of magnetic field lines: magnetic flux density found in nature (usually less than 1 mT)

3) Evaluation procedures

After being stored in a thermostatic chamber (30°C) for 2 weeks, the test samples were measured for the content of associated G-CSF by gel filtration chromatography (n=3).

4) Results

Table 3 shows the content of associated G-CSF after storage at 30°C for 2 weeks.

Table 3

| Effects of magnetic field lines on formation behavior of associated G-CSF | | |
|---|---|---|
| Storage conditions | Content of associated G-CSF | |
| | Average | Standard deviation |
| In the presence of magnetic field lines | 1.5% | 0.04% |
| In the absence of magnetic field lines | 2.3% | 0.06% |

As shown in the results above, the magnetic field lines were found to have an effect of suppressing the formation of associated matter during storage of G-CSF solutions.

**Claims**

1.   A method for stabilizing a protein solution formulation, which comprises storing the protein solution formulation under magnetic field lines.

2.   The method according to claim 1, wherein the magnetic flux density is 1 mT (millitesla) or more.

3.   The method according to claim 1 or 2, wherein the protein is a physiologically active protein.

4. The method according to claim 3, wherein the physiologically active protein is selected from an antibody, an enzyme, a cytokine and a hormone.

5. The method according to claim 4, wherein the physiologically active protein is a hematopoietic factor.

6. The method according to claim 5, wherein the hematopoietic factor is erythropoietin or granulocyte colony-stimulating factor.

7. The method according to claim 4, wherein the physiologically active protein is an antibody.

8. The method according to claim 1, wherein the protein solution formulation is in the form of a pre-filled syringe formulation.

9. A storage container for holding a protein solution formulation, which comprises a magnetic field generator.

10. The storage container according to claim 9, wherein the magnetic field generator is a magnet.

11. A method for stabilizing a protein-containing solution, which comprises storing the protein-containing solution under magnetic field lines.

12. The method according to claim 11, wherein the protein-containing solution is a bulk solution for protein production.

13. A stabilized protein formulation, which is prepared from a bulk solution for protein production stored under magnetic field lines.

14. The formulation according to claim 13, wherein the stabilized protein formulation is in the form of a solution formulation.

15. The formulation according to claim 13, wherein the stabilized protein formulation is in the form of a lyophilized formulation.

16. A method for suppressing the formation of associated matter in a protein solution formulation, which comprises storing the protein solution formulation under magnetic field lines.

17. The use of a magnetic field generator for stabilization of a protein solution formulation.

Figure 1

magnet

Figure 2

b

magnet

a

Figure 3

magnet

Figure 4

magnet

# EP 1 541 165 A1

**INTERNATIONAL SEARCH REPORT**

| International application No. |
|---|
| PCT/JP03/10754 |

| A. CLASSIFICATION OF SUBJECT MATTER |
|---|
| Int.Cl$^7$ A61K38/00, A61K9/08, A61J1/00 |

According to International Patent Classification (IPC) or to both national classification and IPC

| B. FIELDS SEARCHED |
|---|

Minimum documentation searched (classification system followed by classification symbols)
Int.Cl$^7$ A61K38/00, A61K9/08, A61J1/00

Documentation searched other than minimum documentation to the extent that such documents are included in the fields searched

Electronic data base consulted during the international search (name of data base and, where practicable, search terms used)
CAPLUS(STN), MEDLINE(STN), BIOSIS(STN)

| C. DOCUMENTS CONSIDERED TO BE RELEVANT |
|---|

| Category* | Citation of document, with indication, where appropriate, of the relevant passages | Relevant to claim No. |
|---|---|---|
| X<br>A | WO 02/17957 A1 (Chugai Pharmaceutical Co., Ltd.),<br>07 March, 2002 (07.03.02),<br>Particularly, Claims<br>& EP 1329224 A1 | 13-15<br>1-12,16,17 |
| X<br>A | WO 01/17542 A1 (Chugai Pharmaceutical Co., Ltd.),<br>15 March, 2001 (15.03.01),<br>Particularly, Claims<br>& EP 1232753 A1          & JP 2001-521332 A | 13-15<br>1-12,16,17 |
| X<br>A | WO 01/64241 A1 (Chugai Pharmaceutical Co., Ltd.),<br>07 September, 2001 (07.09.01),<br>Particularly, Claims<br>& EP 1260230 A1          & JP 2001-563138 A<br>& US 2003/92622 A1 | 13-15<br>1-12,16,17 |

| ☒ Further documents are listed in the continuation of Box C. | ☐ See patent family annex. |
|---|---|

| * | Special categories of cited documents: | | |
|---|---|---|---|
| "A" | document defining the general state of the art which is not considered to be of particular relevance | "T" | later document published after the international filing date or priority date and not in conflict with the application but cited to understand the principle or theory underlying the invention |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 November, 2003 (19.11.03) | 09 December, 2003 (09.12.03) |

| Name and mailing address of the ISA/<br>Japanese Patent Office | Authorized officer |
|---|---|
| Facsimile No. | Telephone No. |

Form PCT/ISA/210 (second sheet) (July 1998)

12

**EP 1 541 165 A1**

<table>
<tr><td colspan="3">INTERNATIONAL SEARCH REPORT</td><td>International application No.<br>PCT/JP03/10754</td></tr>
</table>

C (Continuation).   DOCUMENTS CONSIDERED TO BE RELEVANT

| Category* | Citation of document, with indication, where appropriate, of the relevant passages | Relevant to claim No. |
|---|---|---|
| X<br>A | WO 00/51629 A1 (Chugai Pharmaceutical Co., Ltd.),<br>08 September, 2000 (08.09.00),<br>Particularly, Claims<br>& JP 2000-247903 A     & EP 1197221 A1 | 13-15<br>1-12,16,17 |
| X<br>A | EP 178665 A2 (Chugai Pharmaceutical Co., Ltd.),<br>15 May, 1986 (15.05.86),<br>Particularly, Claims<br>& JP 61-97229 A     & US 4806524 A | 13-15<br>1-12,16,17 |
| A | JP 2000-229815 A (Toshimitsu HATTORI),<br>22 August, 2000 (22.08.00),<br>(Family: none) | 1-17 |
| A | JP 2001-340062 A (Kabushiki Kaisha Toyo Shin'yaku),<br>11 December, 2001 (11.12.01),<br>(Family: none) | 1-17 |
| A | US 5584994 A (Toshimitsu HATTORI, Masaru OTA),<br>17 December, 1996 (17.12.96),<br>& JP 8-197064 A     & JP 8-197066 A<br>& EP 713842 A3 | 1-17 |
| A | JP 1-262988 A (Kabushiki KaishaShoko Kagaku Kenkyusho),<br>19 October, 1989 (19.10.89),<br>(Family: none) | 1-17 |

Form PCT/ISA/210 (continuation of second sheet) (July 1998)